# EUROPEAN PATENT SPECIFICATION

(11) **EP 2 338 552 B1**
(45) Date of publication and mention of the grant of the patent: **19.07.2017**
(21) Application number: 09016040.9
(22) Date of filing: 28.12.2009
(51) Int. Cl.: A61M 5/172, A61M 5/142, A61M 5/00

(54) **Ambulatory infusion device with bolus administration delay**
Tragbare Infusionsvorrichtung mit verzögerter Bolusverabreichung
Dispositif de perfusion ambulatoire avec temporisation d'administration de bolus

(43) Date of publication of application: 29.06.2011
(73) Proprietor: F. Hoffmann-La Roche AG, 4070 Basel (CH); Roche Diabetes Care GmbH, 68305 Mannheim (DE)
(72) Inventor: Sigrist, Reto, 3207 Golaten (CH); Bernini, Nicole, 3423 Ersingen (DE); Remde, Axel, 3432 Lützelflüh-Goldbach (CH)
(74) Representative: Rentsch Partner AG

(56) References cited:
- WO-A2-03/053498
- WO-A2-2007/056592
- US-A1- 2008 125 701
- US-A1- 2009 177 147

## Description

The invention relates to ambulatory insulin infusion systems as used in the therapy of Diabetes Mellitus by CSII (CSII: Continuous Subcutaneous Insulin Infusion).

Ambulatory insulin infusion systems are used in increasing number for the therapy of Diabetes Mellitus. Those systems include an insulin pump which is designed to be carried by a Person with Diabetes (PwD) substantially continuously night and day and to administer insulin into the PwD's subcutaneous tissue via a subcutaneous cannula, which is replaced by the PwD every few days. Insulin pumps administer insulin according to a basal administration profile which is typically pre-programmed and varies over the time of day. In addition, insulin pumps are designed to administer larger insulin boli on demand for compensating for the intake of food comprising carbohydrates and to lower undesirably raised blood glucose values. A state-of-the-art insulin pump is disclosed, for example, in WO 2003053498 A2 to which reference is made for a typical design and typical features of such devices according to the state of the art.

The US 2009177147 A1 discloses, in an embodiment, an insulin pump into which a meal time may be scheduled or programmed. The insulin pump has a controller with an insulin timing module. If the blood glucose information indicates that the blood glucose level of the user is low, the insulin timing module may delay delivery of a meal bolus of insulin.

The US 2008125701 A1 discloses, in an embodiment, an insulin pump with an acceleration sensor. The insulin pump may be designed to reduce the (basal) insulin administration for exercise or physical activity of a user. An acceleration sensor may be present to detect an end of the exercise or physical activity and the insulin pump may automatically return to a normal programmed delivery rate a time delay after the cessation of the exercise or physical activity.

The WO 2007056592 discloses, in an embodiment, an further insulin pump that is designed to determine a bolus amount based on foodstuff information.

Discreetness of an ambulatory infusion system is known to be a critical factor for the acceptance of CSII by many PwDs who want to draw as little attention as possible, if any, towards their disease. Therefore, modern insulin pumps are designed to be carried concealed from view, e.g. in a trousers pocket, in a holster at a belt or as adhesive patch which is directly attached to the skin. The achievable discreetness for operating and programming of the devices, however, is limited because they are often carried at locations where they are not easily accessible and are coupled to a subcutaneous cannula via an infusion tubing. The programming of a meal bolus prior to a meal is especially critical in this respect since it typically involves additionally measuring a blood glucose value and because meals are often taken in a public environment.

To aid the reader's understanding, the typical procedure for programming a bolus is described in the following for a commercially available state-of-the-art system. This system allows comparatively discreet operation because it includes, besides an insulin pump, a separate diabetes manager with an integrated blood glucose measurement module and allows control over most functions of the pump wirelessly via an RF (RF: radio frequency) interface. The diabetes manager has a size and look similar to an everyday-life electronic device such as a cell phone. In contrast to an insulin pump which shall generally be as small as possible and accordingly only provides a limited user interface, the diabetes manager provides a larger and more comfortable user interface. It can accordingly be operated in a comparatively discrete and convenient way.

When a PwD intends to take a meal, he or she typically performs the following steps (A) - (D):
(A) Taking a blood glucose measurement. This includes the sub-steps of (i) taking a test strip out of a container; (ii) placing the test strip in a corresponding socket of the diabetes manager, thus switching the device on; (iii) piercing the skin with a lancet or piercing device; (iv) placing a drop of blood on a corresponding section of the test strip; (v) reading the measured blood glucose value from a display; (vi) removing and disposing the used test strip.
(B) Marking the measured blood glucose value. For this purpose, the diabetes manager offers a list of situations, such as "before meal", "after meal", "before sports", and the like from which an item may be selected via keys. While this step is not absolutely necessary, it adds valuable information for diary keeping and evaluation purposes and may also be considered in the subsequent step of determining the insulin bolus amount.
(C) Entering the carbohydrate amount of the meal the PwD intends to eat. Alternatively, a meal may be selected from a food database of the diabetes manager. Based on the blood glucose value and the carbohydrate amount, the diabetes manager calculates an appropriate insulin bolus amount for the current time of day. For this purpose, a number of algorithms are known in the art, such as disclosed in the WO 2006/066926.
(D) The calculated bolus amount is displayed on the display of the diabetes manager and is confirmed by the PwD. Optionally, a further step may be carried out in which an appropriate administration profile over time is selected from a list for taking into account varying carbohydrate absorption characteristics of different meals.

After a final confirmation by the PwD, the bolus amount is transmitted to the insulin pump which subsequently administers the bolus.

Depending on the specific system, the details and the sequence of the individual steps may be somewhat different, without deviating from the general procedure.

Carrying out such a sequence of steps cannot be performed in a sufficiently simple and discrete way in a number of situations. For example, in a (public) cantina, a restaurant or the like, the PwD normally would often not be able to pierce the skin and to place a drop of blood on a test strip without drawing undesired interest from others. Furthermore, in some cases, carrying the diabetes manager is simply not convenient during mealtime and directly operating the insulin pump is generally rather indiscreet and/or uncomfortable because it is carried concealed from view.

It is a goal of the present invention to provide ambulatory insulin infusion systems that can be conveniently and discretely operated.

The goal is achieved based on the insight that not all steps which are to be performed by a PwD for administering a bolus have to be carried out immediately at the time when the bolus is to be administered. Instead, some, if not all, of these steps may be carried out in advance of a situation where a bolus shall be administered, thus considerably improving the discreteness and/or comfort for the PwD.

An ambulatory insulin infusion system in accordance with the invention includes:
a) an administration module for insulin administration, the administration module being designed to administer insulin according to a basal profile continuously and additionally a bolus on demand, the bolus having a bolus amount, and
b) a data collection module for collecting data relevant for the bolus amount, the data collection module being operatively coupled to the administration module.

In accordance with the invention, the system further includes a delay module, the delay module being designed to trigger a start of the bolus administration a therapeutically significant delay interval after the collection of the data.

The delay module can be realized using any combination of hardware or software and may especially be integral with the general control circuitry of the system which is typically based on one or multiple microcontrollers running a corresponding firmware code and supplementary circuitry. As will be discussed in more detail below, the delay module may be realized by a timer that is started after the collection of the data and provides a trigger signal for triggering the start of the bolus administration upon expiry of the delay interval. Alternatively, the delay module may be realized by circuitry or firmware code that continuously tests if a condition for terminating the delay interval is met and provides a trigger signal upon the condition being met. A condition for terminating the delay interval may especially be given by the reception of an acknowledgement user input and/or by a signal provided by a continuous glucose measurement unit.

Here and in the following, the term "bolus" generally refers to a meal bolus that is administered in order to compensate the intake of carbohydrates. The equivalent term "meal bolus" may be used in order to avoid any ambiguity. A bolus that is administered to lower an undesirably raised blood glucose value is explicitly referred to as "correction bolus."

The phrase "therapeutically significant delay interval" refers to a delay interval that has a non- negligible impact on the PwD's blood glucose curve. That is, the resulting blood glucose curve over time is significantly different as compared to an administration of the bolus without the delay interval. The minimum length of a delay interval to be therapeutically significant is typically in the range of some minutes, in dependence of the PwD and the absorption characteristics of the insulin formulation. A delay in the order of some seconds as it may be present, e.g., in state-of-the art devices between the programming of a bolus by the PwD and the beginning of its administration is not considered as therapeutically significant.

For the following description, it is generally assumed that the administration module and the data collection module are made or included by separate physical units and are included in separate housings. The unit including the administration module is referred to as the "insulin pump" and the unit including the data collection module is referred to as the "diabetes manager". This overall architecture, which is also present in some commercially available systems, however, is not essential. The system may alternatively be provided in form of a single device with a single housing or include a larger number of separate devices.

With current systems, some PwDs program and administer a bolus some time prior to the meal for discreetness reasons. In this case, however, the insulin acts already prior to the meal, leading to a period of uncontrolled and generally too low blood glucose level, sometimes involving severe complications such as a hypoglycemia between administration of the bolus and eating the meal, which may take place for example 30 or more minutes later. Other PwDs program and administer the bolus only some time after having finished the meal, e.g., back in their office after lunch, or in the restroom of a restaurant. This however, is accompanied by the drawback that the insulin is generally administered too late and is even forgotten in some cases, resulting in undesired hyperglycemic episodes.

A system in accordance with the invention allows to carry out most, if not all, operational steps at a place and time that is convenient for the PwD some time prior to the meal while the actual administration is performed later on at the end of the delay interval, thus avoiding the above-described drawbacks or complications.

The PwD can, for example, perform a blood glucose measurement and further operational steps as described above in his or her office prior to going to lunch or in the restroom of a restaurant after ordering the meal. Thereafter, the bolus is administered when actually required, namely when the user actually sits down for lunch or the meal is actually served in the restaurant.

In some embodiments, the system includes a bolus determination module, the bolus determination module being designed to determine the bolus amount based, at least in part, on the collected data.

In this context, collected data relevant for a meal bolus amount especially include a carbohydrate amount of the meal and may additionally include further data such as the relative amounts of fat, carbohydrates and proteins, as well as information about a potential sickness which tends to increase the insulin demand, the amount and/or level of sportive activity which tends to reduce the insulin demand, and the like.

Determining the bolus amount may be carried out when all required or desired data are collected. In an embodiment including an insulin pump and a diabetes manager, the bolus determination module may be included in the diabetes manager and the determined bolus amount and further data required for the administration are subsequently transmitted to the insulin pump.

As will be discussed below, some data may only be available at the time when the bolus shall actually be administered. In some embodiments, the diabetes manager does further not need to be present when the bolus is actually administered at the end of the delay interval. Therefore, the data collection module may, fully or in part, be integral with the insulin pump and the bolus determination unit may, fully or in part, be integral with the insulin pump.

In some embodiments including a bolus determination module, the data collection module includes a user interface, the user interface being designed to collect meal characterizing data from a PwD, and the bolus determination module is designed to determine the bolus amount based, at least in part, on the meal characterizing data. The meal characterizing data may be entered directly or recalled from a food database, the food database storing the corresponding meal characterizing data for a variety of meals. In embodiments including a diabetes manager and an insulin pump as separate physical devices which are operatively coupled as described above, the corresponding user interface may be provided on either or both of the devices. The user interface may be realized by a number of pushbuttons and a display, but may also include a touch screen, a track ball, a voice recognition unit, or the like.

Determining the bolus amount typically involves time-varying and/or patient-dependent parameters, such as carbohydrate factors, which are stored by the bolus determination module. For a delayed bolus administration, those parameters may be considered either for the time of day when the determination is carried out or for the time of day when the bolus shall actually be administered.

Alternatively or additionally to determining a bolus amount via a bolus determination module, the user interface may be designed for direct entry of the bolus amount, e.g., as a numeric value entry or via scroll buttons, which is still preferred by some PwDs. In this case, the collected data are identical with or comprise the bolus amount.

In some embodiments, the data collection module includes a blood glucose measurement module and the bolus determination module is designed to determine the bolus amount based, at least in part, on a blood glucose value measured by the blood glucose measurement module. Reducing the bolus amount is favorable if the current blood glucose value is particularly low. In contrast, increasing the bolus amount is favorable if the blood glucose value is particularly high. The blood glucose measurement module may, for example, be a strip-based optical or electrochemical measurement module, but may also be a continuous blood glucose meter.

In some embodiments, the bolus determination module is designed to determine, based on a measured blood glucose value, a correction bolus amount of a correction bolus and the administration module is designed to trigger a start of correction bolus administration without therapeutically significant delay. Since a correction bolus is administered in order to lower an undesirably raised glucose value, it may be administered immediately after being determined without a preceding delay interval. Alternatively, the correction bolus may be administered along with the meal bolus after the delay interval.

In some embodiments, the delay module is designed to trigger the start of the bolus administration after a pre-defined time interval, the pre-defined time interval defining the delay interval. After termination of the delay interval, the administration may be carried out automatically without further user input or after some further user input as will be discussed below.

This type of embodiment is especially useful if the appropriate delay interval is already known when the data relevant for a bolus amount are collected by the system. An exemplary scenario is if a PwD goes to lunch knowing there is a constant or negligible time before the PwD will begin eating. The length of the pre-defined time interval may be entered via a user interface e.g. when performing the steps of measuring the blood glucose value, providing food characterizing data, and the like, as described above. Alternatively or additionally, it may be selected from a list of pre-defined delay intervals or may be a fixed time interval. While there are no fixed limits for longest or shortest delay interval, the shortest possible delay interval may, for example, be in the range of 5min to 10min and the longest delay interval may, for example, be in the range of 30min to 2h. For triggering the bolus administration after a pre-defined delay interval, the delay module may comprise a corresponding delay timer that is started following the data collection.

In some embodiments, the delay module is designed to receive, in the delay interval, an acknowledgement user input and to trigger the start of the bolus administration upon reception of the acknowledgement user input. This type of embodiment is especially useful if the appropriate delay interval is not known in advance. In a restaurant, for example, the waiting time may vary considerably and be difficult to estimate in advance. This type of embodiment allows the PwD to carry out all or most of the steps that may be carried out in advance and can typically not be performed in a discrete way in advance but to initiate the bolus administration only on demand. This step may, for example, be carried out by a single key press in a discrete way since no complex action such as watching a display or entering numeric values is required here. In embodiments including different separate devices, the corresponding input device for providing the acknowledgement user input may be included by any of the devices. In some embodiments, it is included by the same unit as the administration module, e.g. an insulin pump, since it is always carried by the PwD. The input device, e.g. a push button, may be designed for tactile feeling, e.g. through clothes. As described below in more detail, the system may repeatedly remind the PwD about the active delay interval in intervals of, e.g., 1 min or 3min.

In some embodiments, the system includes or is operatively coupable to further electronic devices, such as a cell phone or a smart pone, e.g. via a Bluetooth RF interface. In this case, a user interface of those devices may be used for providing the acknowledgement user input which is subsequently transmitted to the insulin pump or diabetes manager.

In some embodiments including triggering the start of the bolus administration upon reception of an acknowledgement user input, the delay module is designed to cancel a running delay interval without triggering the bolus administration if a maximum delay interval has expired without reception of the acknowledgement user input.

Such an embodiment is useful, for example, if the PwD is disrupted by a colleague before going to lunch and during a long discussion with the colleague the blood glucose measurement may become a wrong basis for calculating a meal bolus amount. Hence, if the user acknowledgement input is not provided within a predefined time interval of, for example 30min or 45min, the delay interval is cancelled and the bolus is not administered. The PwD is therefore forced to repeat the blood glucose measurement.

Alternatively, the delay interval is not cancelled in such a situation. Instead, the PwD is informed about the long delay, for example with an optical, acoustic and/or tactile signal, such that the PwD can decide on his or her own if the meal bolus shall be administered. If the PwD did have an intensive physical activity since the blood glucose measurement, he or she may decide to repeat the measurement. If no exceptional activities were performed since the last blood glucose measurement, the PwD may decide not to repeat the measurement and administer the meal bolus irrespective of the fact that the delay interval has exceeded a maximum delay interval.

In some embodiments, the system includes an alert module in order to generate an alert while the alert interval is running and/or when the delay interval is terminated.

The alert may comprise any of an acoustical, an optical or a tactile signal. Tactile signals allow the alert to be especially discrete. The alert module may be included in the same physical unit as the administration module since it is generally carried by the PwD. The user may be alerted regularly, for example in intervals of between 2min and 6min while the delay interval is running.

In embodiments where the administration is automatically carried out after a pre-defined delay interval, providing an alert when the delay interval is terminated serves as confirmation for the PwD that the bolus will now be administered. Optionally, the PwD may have the possibility to prevent the bolus administration. This may be useful, for example, in a situation where the PwD is hindered from actually taking the meal.

In some embodiments, the delay module can be selectively disabled. This type of embodiment is especially suited for PwDs which require or wish a delayed bolus administration only occasionally. Alternatively or additionally to explicitly disabling the delay module, the delay interval may be set to zero in this case. Enabling or disabling of the delay module may be carried out by the user, e.g., along with providing food characterizing data and/or measuring the blood glucose value as described above.

In accordance with an overall concept of the invention, the amount of operational steps that have to be carried out by the diabetic immediately when a bolus shall be administered should be minimized, if not fully avoided.

In some situations, however, some of the information that is required for determining the bolus amount may be available only immediately when the bolus shall be administered. For example, such data may relate to the size of the meal, which the PwD did not specify yet or which was served as a larger or smaller portion than expected. The PwD may also wish to change the type of meal, if, for example, a salad is served instead of a soup as a starter.

In some embodiments, the system is therefore designed to receive, at the end of the delay interval, further data relevant for the determination of the bolus amount and to determine the bolus amount, at least in part, based on the further data. The further data may be new data and/or an adjustment of previously entered data.

These further data relevant for the determination of the bolus amount can be entered using the diabetes manager and/or the insulin pump. Using the diabetes manager is preferable if a large amount of data like different meal types and/or meal sizes have to be entered, as the diabetes manager usually has a comfortable user interface. If only a small amount of data has to be entered, using the insulin pump may be preferable since it does not require carrying the diabetes manager.

In embodiments of the system including or being operatively coupable to another electronic device such as a cell phone, the system may be designed such that the other device can be used for inputting the further data in an especially discrete manner.

In embodiments that allow providing further data at the end of the delay interval, a bolus determination unit may be included, fully or in part, in the insulin pump. Including the bolus determination unit, at least in part, in the insulin pump is further favorable if computation parameters such as carbohydrate ratios shall be considered for the time of day when the administration is actually carried out and the delay interval is not known in advance as it is the case for some embodiments as described above.

In some embodiments where the system is split into separate devices, in particular an insulin pump and a diabetes manager, the delay module is favorably arranged in the same device as the administration module. This type of embodiment has the particular characteristic that no communication of the insulin pump with other units or devices is required when the delay interval is terminated and the bolus is administered. Alternatively, the delay module may be integral with another device, in particular a diabetes manger. In this case, the diabetes manager transmits a corresponding signal to the insulin pump at the end of the delay interval, thus causing the insulin pump to start the bolus administration.

In some embodiments, the system includes or is operatively coupable to a continuous glucose monitoring unit and is designed to take into account blood glucose values measured at the beginning and/or during and/or at the end of the delay interval for determining the bolus amount.

Continuous glucose measurement devices are increasingly used by PwDs in order to tightly control their blood glucose level. Such devices may be integrated with or operatively coupled to a system in accordance with the invention. In embodiments which consider the blood glucose value for determining the bolus amount, the readings of a continuous measurement system may be used in addition or alternatively to a glucose measurement before the start of the delay interval in order to allow the consideration of blood glucose changes in the delay interval.

In some embodiments, a continuous glucose monitoring unit is used additionally or alternatively to detect a blood glucose raise resulting from a carbohydrate intake and the delay module is configured to trigger the start of the bolus administration upon the detection of the blood glucose raise. This type of embodiment is an alternative to providing a manual acknowledgement user input as described above. Instead, the fact that a carbohydrate intake is associated with a blood glucose increase is utilized for triggering the start of the bolus administration.

Besides convenience and discreteness as discussed so far, the invention is of particular use for PwDs which additionally suffer from gastroparesis. Those persons have to generally administer a meal bolus with some patient-specific delay as compared to the food intake. Such delayed administration however, is known to be occasionally forgotten. In accordance with the invention, the PwD may carry out all steps and input all data at the time of the meal intake in the same way as carried out by a PwD not suffering from gastroparesis with. The delay interval is used to delay the actual administration as required in accordance with the gastro paresis. For these applications, embodiments that allow automatic administration after termination of a pre-defined delay interval as described above are especially well-suited. If a PwD suffering from gastroparesis whishes an additional delay interval for discreetness reasons, the corresponding delay interval may be added. If the invention is used for accommodating gastroparesis, an alert at the termination of the delay interval may not be necessary and/or desirable.

In some embodiments, the delay module is designed to store a minimum delay interval, the minimum delay interval being a lower limit for the length of the delay interval. The minimum delay interval may especially be the delay interval required because of the PwD's gastroparesis.

A minimum delay interval may be implemented in different ways. For example, a single delay timer as described above may be present in the delay module with a preset minimum delay interval. Alternatively, a first delay timer for the minimum delay interval and a second delay timer for a further delay interval are provided, with the termination of the first delay interval triggering the beginning of the second delay interval. In embodiments where the delay module is designed to receive an acknowledgement user input and to terminate the delay interval upon reception of the acknowledgement user input, the delay module may be designed not to accept or react on the acknowledgement user input unit termination of the minimum delay interval. In this way, a minimum delay in accordance with the PwD's gastroparesis requirements is ensured.

In the following, exemplary embodiments of systems in accordance with the invention as well as methods for the usage of such systems are described in more detail with reference to the figures.
Fig. 1 shows a diabetes manager in accordance with the invention.
Fig. 2 schematically shows the major units and the architecture of a system, including a diabetes manager and an insulin pump in accordance with the invention.
Fig. 3 shows an operational flow diagram for a bolus administration in accordance with an exemplary embodiment of the invention.
Fig. 4 shows an operation flow diagram for a bolus administration in accordance with a further exemplary embodiment of the invention.

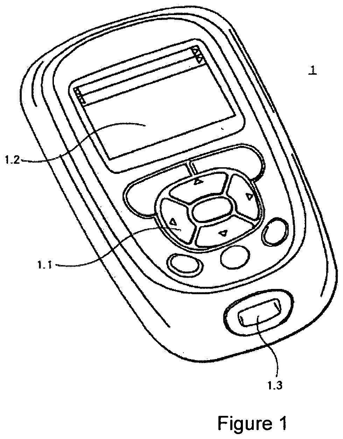

Fig. 1 shows a diabetes manager 1 in accordance with the invention. The diabetes manager 1 includes a keypad 1.1, a display 1.2, and a blood glucose measurement module with a test strip socket 1.3. The diabetes manager 1 typically includes one or multiple microcontrollers running a corresponding firmware code as well as supplementary circuitry.

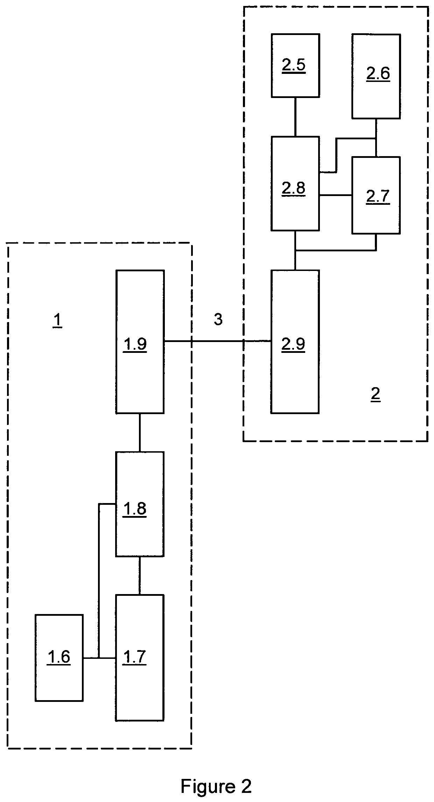

Figure 2 schematically shows a block diagram of an insulin infusion system including a diabetes manager 1 and an insulin pump 2.

The display 1.2 and the keypad 1.1 as well as an optional acoustic and/or tactile indictor, in combination, form the user interface 1.6 of the diabetes manager 1. The blood glucose measurement module 1.7 is typically a strip-based electrochemical or optical blood glucose measurement module. The blood glucose measurement module 1.7 and the user interface 1.6, in combination, serve as data collection module.

A bolus determination module 1.8 is arranged in the diabetes manager 1 to determine meal bolus amounts and optionally correction bolus amounts based on the collected data as described above.

The insulin pump 2 includes an administration controller 2.8, a delay module 2.7, a user interface 2.6, and a pump module 2.5. The administration controller 2.8 and the pump module 2.5, in combination, form the administration module. The user interface 2.6 typically includes a display, a number of input units such as keys and an alert module with an acoustic and/or a tactile indicator, such as a pager vibrator.

Both the diabetes manager 1 and the insulin pump 2 additionally comprise general control circuitry as typical for such devices which is not explicitly shown. Some of the modules shown in Fig. 2, such as the bolus determination module 1.8, the administration controller 2.8, and the delay module 2.7 may be realized, fully or in part, as integral with the general control circuitry and may be implemented by corresponding firmware code.

Both the diabetes manager 1 and the insulin pump 2 comprise corresponding communication interfaces 1.9 and 2.9 for establishing a communication channel 3 between the devices. The communication interfaces are typically designed for wireless communication over a distance up to 1 m to 3m and are designed, for example, as an IR or Bluetooth RF interfaces.

Either or both of the communication interfaces may optionally further be used for data exchange and/or communication with further devices, such as a cell phone or a PC.

Via the communication channel 3, the diabetes manager 1 can transmit data to the insulin pump 2 like a bolus amount to be administered and further data or commands for remotely controlling the insulin pump 2. In the other direction the administration device 2 may transmit an effectively administered bolus amount along with a time stamp to the diabetes manager 1, e.g. for confirmation and record-keeping purposes. Further data may be transmitted between the diabetes manager 1 and the administration device 2 as needed.

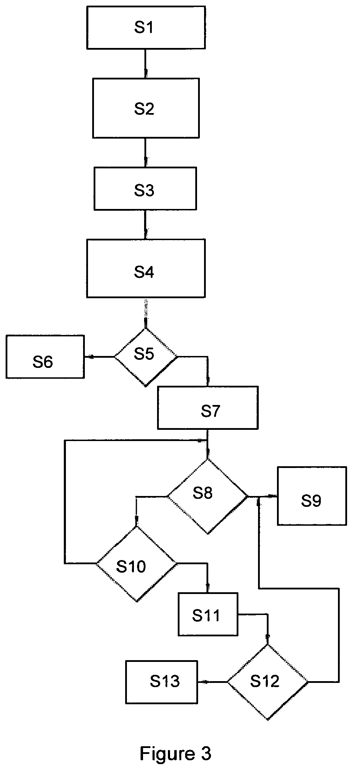

For explaining the operation of an exemplary system, reference is additionally made to Fig. 3 which shows the major steps of the operational flow for a bolus administration.

In step S1, the PwD performs a blood glucose measurement, using the blood glucose measurement module 1.7 and the user interface 1.6. While generally being recommended, the blood glucose measurement is not essential for the invention. A marking of the measured value into situations like "before meal" or "after meal" as explained above (but not reflected in Fig. 3) may optionally be performed.

In step S2, the PwD enters meal characterizing data with respect to the meal he or she is planning to eat. These data typically include the meal size and in particular the amount of carbohydrates. The data may optionally additionally include further data such as the fat and protein content. The entry of these data may be simplified by a food database (not shown) comprised by the diabetes manager 1.

Optionally, an administration profile for the administered bolus amount as a function of time may be selected in order to compensate for different food compositions, in particular for the amount of fat and protein that can have a considerable influence on the carbohydrate absorption rate. If the diabetes manager 1 includes a food database, this information may be stored along with the food type in the database. The default setting is advantageously an administration in a substantially continuous way and in a short time.

Rather than entering data with respect to food in step S2, the PwD may directly enter the bolus amount in this step if he or she prefers to do so.

Based on the food entry data or a bolus amount entered directly in step S2 and perhaps a measured blood glucose value, the determination module 1.8 determines the bolus amount to be administered in step S3. The bolus amount typically has two components: first, a correction bolus amount for lowering an undesirably raised blood glucose value, and second a meal bolus amount for compensating for the coming meal intake.

The determined bolus amount may be displayed to the PwD, for example using the display 1.2 of diabetes manager 1, and the PwD may have the possibility to adjust the determined bolus amount to a desired value if he or she judges the determined bolus amount as inappropriate for any reason.

Besides the blood glucose value and the meal characterizing data, the determination may consider additional factors, in particular, the amount of currently active insulin in the PwD's body (Insulin on Board), resulting from prior administrations.

In addition to the data discussed so far, the PwD enters via the user interface 1.2 whether the bolus shall be administered immediately or after a delay interval.

Via the communication channel 3, all data required by the insulin pump 2 for carrying out the administration are transmitted from the diabetes manager 1 to the pump 2 in step S4.

All steps S1 to S4 may be carried out by the PwD in a private environment, for example, in his or her office or a restaurant restroom. After the data transmission in step S4, the diabetes manager 1 is not required for the subsequent steps. So, the diabetes manager 1, may, e.g., be left and not be taken along to where the meal is to be served.

After the data transmission from the diabetes manager 1 to the insulin pump 2, the further operational flow branches in step S5 in dependence on whether the meal bolus shall be administered immediately or delayed. For immediate administration, the delivery of both the meal bolus and possibly a correction bolus is carried out immediately in step S6 as in state-of-the-art devices. This would be a typical situation, e.g., if the PwD is at home and special discreetness is not required.

If the meal bolus shall be administered after a delay interval, a correction bolus is advantageously administered without delay in step S7 because the insulin for correcting a raised glucose value should generally be administered as soon as possible. The following operational steps are carried out under control of the delay module 2.7.

In step S8 it is checked whether the delay interval shall be terminated and the bolus administration shall be triggered. Administration of the meal bolus may be triggered via the user interface 2.6 of the insulin pump 2, for example by a single key press, thus providing the acknowledgement user input for triggering the start of the administration. Alternatively or additionally, the acknowledgement user input may be provided via the diabetes manager 1. In this case, the acknowledgement user input is transmitted from the diabetes manager 1 to the insulin pump 2 via the communication channel 3. Reception of the acknowledgement user input is advantageously notified, e.g., by an acoustic and/or tactile indication, via the user interface 2.6 of the administration device. In step S9, the administration of the bolus amount is carried out by the pump module 2.5 under control of the administration controller 2.8.

If the administration is not triggered and the delay interval is accordingly not terminated, it is checked in an optional step S10 if a timeout has occurred, that is, if a maximum delay interval has expired. The maximum delay interval may be a user-selectable parameter and/or may be a preset value. The maximum delay interval may be in a range of about 30min to 180min A maximum delay interval of 45 or 60min is believed to be appropriate in many cases. Other values, however, may be used as well. The maximum delay interval ensures that a meal bolus is not administered if the general circumstances have considerably changed as compared to the time of its determination, as was explained above.

If a timeout occurs, the PwD is advantageously alerted in step S11 via the alert module of the user interface 2.6 and may decide to immediately trigger the bolus administration in step S12. In this case, the administration is carried out in step S9. This situation may occur, for example, if the PwD forgets to actually trigger the administration when eating the meal. If the PwD does not trigger the administration in step S12, the delay interval is canceled in step S13. Alternatively, the operational flow may directly proceed with step S13 and cancel the delay interval in the case of a timeout.

If no timeout is detected in step S10, the operational flow proceeds with step S8. In this case, the steps S8 and S10 are carried out in a continuous cycle as long as no acknowledgement user input is provided and no timeout has occurred.

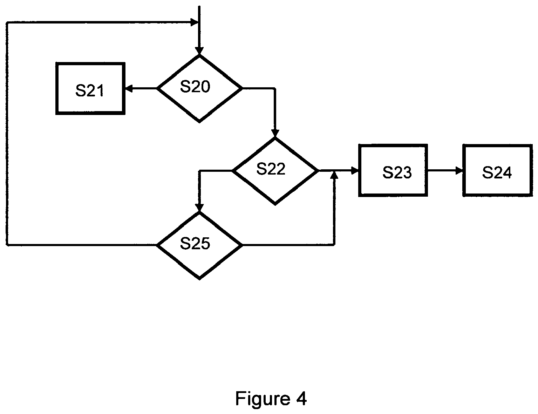

Figure 4 illustrates the operational flow for a further exemplary embodiment. For the general architecture of the system, reference is made to Figure 2. The operation of some units and in particular the delay module 2.7, however, is somewhat different as described in the following.

For this type of embodiment, the first steps of the operational flow are largely identical to steps S1 to S7 of the previously described embodiment and as shown in Figure 3. However, along with entering food characterizing data and/or further data relevant for the bolus amount, the PwD enters a time interval via the user interface 1.6 or selects a desired time interval from a list. The time interval subsequently serves as pre-defined delay interval. The delay interval may be zero in situations where the PwD wishes an administration without delay.

Figure 4 shows the operational steps following step S7 of Figure 3. In step S20, it is tested if the delay interval shall be cancelled. Canceling the delay interval may be carried out via a corresponding user input through the user interface 2.6 of the insulin pump 2. Alternatively or additionally, it may be carried out via a user input on the user interface 1.6 of the diabetes manager 1 or via a user input on a further device, such as a cell phone, which is operatively coupled to the system via either of the communication interfaces 1.9, or 2.9 or a further communication interface. If the PwD cancels the delay interval, the operational flow for the bolus administration is terminated in step S21 without the administration being carried out. As described above, the option for cancelling the delay interval is useful in situations where the PwD, for some reason, does not whish the bolus to be administered.

If the delay interval is not canceled in step S20, it is tested in step S22 if the PwD has decided to manually terminate the delay interval and trigger the administration, even though the fixed delay interval has not yet expired. This option is useful for example in a situation where the PwD has programmed a delay interval, for example in accordance with his experience in a restaurant, but the meal is served earlier than expected. In an analogue way to canceling the delay interval and preventing the bolus administration, the user input for carrying out the administration may be provided via either of the user interfaces 1.6, 2.6 of the diabetes manager 1 or the insulin pump 2, respectively, or via a further external device.

If the PwD decides that the administration shall be carried out, he or she may, in step S23, enter further data that are relevant for determining the bolus amount or adjust previously provided data as described above. If such further data are provided or adjusted, step S23 further includes determining an updated bolus amount. For this purpose, the insulin pump 2 comprises a bolus determination unit in addition to the bolus determination unit 1.7 of the diabetes manager 1. The bolus determination unit of the insulin pump 2 may offer the same functionality as the bolus determination unit 1.6 of the diabetes management device 1. It may, however, also offer different and in particular limited functionality. Determining correction bolus amounts, for example, may be carried out only by the bolus determination unit 1.6 of the diabetes manager.

In a further option, the diabetes manager 1 does not include a bolus determination unit and the bolus determination is generally carried out on the insulin pump 2. For this type of embodiment, meal characterizing and further data as described above are transmitted from the diabetes manager 1 to the insulin pump 2 rather than a bolus amount.

In step 24, the bolus administration is triggered.

If the PwD does not decide to terminate the delay interval in step S22, it is tested in step S25 if the pre-defined delay interval has expired. In this case, the operational flow continues with the potential entry of further data in step S23 and the administration in step S24. Otherwise, the operational flow continues with step S20.

For this type of embodiment, the steps S20, S22, and S25 are carried out in a continuous cycle unless the delay interval is canceled or manually terminated via a user input or the pre-defined delay interval has expired.

Alternatively or additionally to improving the discreetness, embodiments with a pre-defined delay interval are of particular use for PwDs which additionally suffer from gastroparesis as discussed above. The pre-defined delay interval is the delay interval that is required because of the gastroparesis or a longer delay interval if the PwD whishes an additional delay of the administration for discreetness reasons.

From the exemplary embodiments, further embodiments may be derived by a person skilled in the art. For example, in the embodiment as illustrated in Figure 4, some or all of the steps S20, S21, S22, S23 may, fully or partly, be omitted. In such an embodiment, step S25 of testing if the fixed delay interval has terminated is carried out continuously and the administration is triggered in step S24 once this condition is fulfilled.

In a further variant, no bolus determination unit 1.6 is present and the PwD directly enters the bolus amount of a bolus to be administered.

In the described embodiments, the diabetes manager 1 and the insulin pump 2 are separate devices. This separation, however, is not essential. All data entry and the determination of the bolus amount may also be carried out directly on the insulin pump 2.

## Claims

1. Ambulatory insulin infusion system, including:
a) an administration module (2.5, 2.8) for insulin administration, the administration module (2.5, 2.8) being designed to administer insulin according to a basal profile continuously and additionally a bolus on demand, the bolus having a bolus amount,
b) a data collection module (1.1, 1.2, 1.3, 1.6, 1.7) for collecting data relevant for the bolus amount, the data collection module (1.1, 1.2, 1.3, 1.6, 1.7) being operatively coupled to the administration module (2.5, 2.8),
c) a delay module (2.7), the delay module (2.7) being designed to trigger a start of the bolus administration a therapeutically significant delay interval after the collection of the data, **characterized by** the delay module being realized by circuitry or firmware code that continuously tests if a condition for terminating the delay interval is met and provides a trigger signal upon the condition being met, the condition for terminating the delay interval being given by the reception of an acknowledgement user input and/or by a signal provided by a continuous glucose measurement unit.

2. Ambulatory insulin infusion system according Claim 1, **characterized by** a bolus determination module (1.8), the bolus determination module being designed to determine the bolus amount based, at least in part, on the collected data.

3. Ambulatory insulin infusion system according Claim 2, **characterized by** the data collection module (1.1, 1.2, 1.3, 1.6, 1.7) including a blood glucose measurement module (1.7) and the bolus determination module (1.8) being designed to determine the bolus amount based, at least in part, on a blood glucose value measured by the blood glucose measurement module (1.7).

4. Ambulatory insulin infusion system according Claim 3, **characterized by** the bolus determination module (1.8) being designed to determine, based on a blood glucose value measured by the blood glucose measurement module (1.7), a correction bolus amount of a correction bolus and the administration module (2.7, 2.8) being designed to trigger a start of the correction bolus administration without therapeutically significant delay.

5. Ambulatory insulin infusion system according either of Claim 2 to Claim 4, **characterized by** the data collection module (1.1, 1.2, 1.3, 1.6, 1.7) including a user interface (1.1, 1.2), the user interface (1.1, 1.2) being designed to collect meal characterizing data from a user, and the bolus determination module (1.8) being designed to determine the bolus amount based, at least in part, on the meal characterizing data.

6. Ambulatory insulin infusion system according to any of the preceding claims, **characterized by** the delay module (2.7) being designed to trigger the start of the bolus administration after a pre-defined time interval, the pre-defined time interval defining the delay interval.

7. Ambulatory insulin infusion system according to any of the preceding claims wherein the delay module (2.7) is configured to receive, in the delay interval, an acknowledgement user input, **characterized by** the delay module (2.7) being designed to cancel a running delay interval without triggering the bolus administration if a maximum delay interval has expired without reception of the acknowledgement user input.

8. Ambulatory insulin infusion system according to any of the preceding claims, **characterized by** an alert module (2.2) in order to generate an alert while the delay interval is running and/or when the delay interval is terminated.

9. Ambulatory insulin infusion system according to any of the preceding claims, **characterized in that** the delay module (2.7) can be selectively disabled.

10. Ambulatory insulin infusion system according to any of the preceding claims, **characterized by** the administration module (2.5, 2.8) and the data collection module (1.6) being included in separate housings.

11. Ambulatory insulin infusion system according Claim 10, **characterized by** the delay module (2.7) being arranged in the same housing as the administration module (2.5, 2.8).

12. Ambulatory insulin infusion system according to any of the preceding claims, **characterized in that** the system includes or is operatively coupable to a continuous glucose monitoring unit and is designed to take into account blood glucose values measured at the beginning and/or during and/or at the end of the delay interval for determining the bolus amount.

13. Ambulatory insulin infusion system according to any of the preceding claims, **characterized by** the delay module (2.7) being designed to store a minimum delay interval, the minimum delay interval being a lower limit for the length of the delay interval.

## Patentansprüche

1. Tragbares Insulininfusionssystem, umfassend:
a) ein Verabreichungsmodul (2.5, 2.8) für die Insulinverabreichung, wobei das Verabreichungsmodul (2.5, 2.8) dazu ausgelegt ist, Insulin nach einem Basalprofil kontinuierlich und zusätzlich bei Bedarf einen Bolus zu verabreichen, wobei der Bolus eine Bolusmenge umfasst,
b) ein Datensammelmodul (1.1, 1.2, 1.3, 1.6, 1.7) zum Sammeln von Daten, die für die Bolusmenge relevant sind, wobei das Datensammelmodul (1.1, 1.2, 1.3, 1.6, 1.7) mit dem Verabreichungsmodul (2.5, 2.8) in Wirkverbindung steht,
c) ein Verzögerungsmodul (2.7), wobei das Verzögerungsmodul (2.7) dazu ausgelegt ist, einen Start der Bolusverabreichung mit einem therapeutisch signifikanten Verzögerungsintervall nach Sammlung der Daten auszulösen, **dadurch gekennzeichnet, dass** das Verzögerungsmodul durch eine Schaltung oder Firmware-Code realisiert wird, die bzw. der kontinuierlich prüft, ob eine Bedingung zum Beenden des Verzögerungsintervalls erfüllt ist und ein Auslösesignal bereitstellt, wenn die Bedingung erfüllt ist, wobei die Bedingung zum Beenden des Verzögerungsintervalls durch den Empfang einer bestätigenden Benutzereingabe und/oder durch ein Signal, das von einer kontinuierlichen Glukose-Messeinheit bereitgestellt wird, gegeben wird.

2. Tragbares Insulininfusionssystem nach Anspruch 1, **gekennzeichnet durch** ein Bolusbestimmungsmodul (1.8), wobei das Bolusbestimmungsmodul dazu ausgelegt ist, die Bolusmenge zumindest teilweise auf Basis der gesammelten Daten zu bestimmen.

3. Tragbares Insulininfusionssystem nach Anspruch 2, **dadurch gekennzeichnet, dass** das Datensammelmodul (1.1, 1.2, 1.3, 1.6, 1.7) ein Blutzuckermessmodul (1.7) aufweist und das Bolusbestimmungsmodul (1.8) dazu ausgelegt ist, die Bolusmenge zumindest teilweise auf Basis eines von dem Blutzuckermessmodul (1.7) gemessenen Blutzuckerwerts zu bestimmen.

4. Tragbares Insulininfusionssystem nach Anspruch 3, **dadurch gekennzeichnet, dass** das Bolusbestimmungsmodul (1.8) dazu ausgelegt ist, auf Basis eines von dem Blutzuckermessmodul (1.7) gemessenen Blutzuckerwerts eine Korrekturbolusmenge eines Korrekturbolus zu bestimmen, und das Verabreichungsmodul (2.7, 2.8) dazu ausgelegt ist, einen Start der Korrekturbolusverabreichung ohne therapeutisch signifikante Verzögerung auszulösen.

5. Tragbares Insulininfusionssystem nach einem der Ansprüche 2 bis 4, **dadurch gekennzeichnet, dass** das Datensammelmodul (1.1, 1.2, 1.3, 1.6, 1.7) eine Benutzerschnittstelle (1.1, 1.2) aufweist, wobei die Benutzerschnittstelle (1.1, 1.2) dazu ausgelegt ist, Mahlzeiten charakterisierende Daten von einem Benutzer zu sammeln, und wobei das Bolusverabreichungsmodul (1.8) dazu ausgelegt ist, die Bolusmenge zumindest teilweise auf Basis der Mahlzeiten charakterisierenden Daten zu bestimmen.

6. Tragbares Insulininfusionssystem nach einem der vorhergehenden Ansprüche, **dadurch gekennzeichnet, dass** das Verzögerungsmodul (2.7) dazu ausgelegt ist, den Start der Bolusverabreichung nach einem vordefinierten Zeitintervall auszulösen, wobei das vordefinierte Zeitintervall das Verzögerungsintervall definiert.

7. Tragbares Insulininfusionssystem nach einem der vorhergehenden Ansprüche, wobei das Verzögerungsmodul (2.7) dazu konfiguriert ist, in dem Verzögerungsintervall eine bestätigende Benutzereingabe zu empfangen, **dadurch gekennzeichnet, dass** das Verzögerungsmodul (2.7) dazu ausgelegt ist, ein laufendes Verzögerungsintervall ohne Auslösung der Bolusverabreichung abzubrechen, wenn ein maximales Verzögerungsintervall ohne Empfang der bestätigenden Benutzereingabe verstrichen ist.

8. Tragbares Insulininfusionssystem nach einem der vorhergehenden Ansprüche, **gekennzeichnet durch** ein Warnmodul (2.2) zur Erzeugung einer Warnmeldung, während das Verzögerungsintervall läuft und/oder wenn das Verzögerungsintervall beendet wird.

9. Tragbares Insulininfusionssystem nach einem der vorhergehenden Ansprüche, **dadurch gekennzeichnet, dass** das Verzögerungsmodul (2.7) selektiv deaktiviert werden kann.

10. Tragbares Insulininfusionssystem nach einem der vorhergehenden Ansprüche, **dadurch gekennzeichnet, dass** das Verabreichungsmodul (2.5, 2.8) und das Datensammelmodul (1.6) in getrennten Gehäusen enthalten sind.

11. Tragbares Insulininfusionssystem nach Anspruch 10, **dadurch gekennzeichnet, dass** das Verzögerungsmodul (2.7) im selben Gehäuse angeordnet ist wie das Verabreichungsmodul (2.5. 2.8).

12. Tragbares Insulininfusionssystem nach einem der vorhergehenden Ansprüche, **dadurch gekennzeichnet, dass** das System eine kontinuierliche Glukoseüberwachungseinheit aufweist oder damit in Wirkverbindung gebracht werden kann und dazu ausgelegt ist, Blutzuckerwerte zu berücksichtigen, die zu Beginn und/oder während und/oder am Ende des Verzögerungsintervalls gemessen wurden, um die Bolusmenge zu bestimmen.

13. Tragbares Insulininfusionssystem nach einem der vorhergehenden Ansprüche, **dadurch gekennzeichnet, dass** das Verzögerungsmodul (2.7) dazu ausgelegt ist, ein Mindestverzögerungsintervall zu speichern, wobei das Mindestverzögerungsintervall eine Untergrenze für die Länge des Verzögerungsintervalls ist.

## Revendications

1. Système de perfusion d'insuline ambulatoire, comportant .
a) un module d'administration (2.5, 2.8) pour l'administration d'insuline, le module d'administration (2.5, 2.8) étant conçu pour administrer de l'insuline de façon continue en fonction d'un profil de base et de surcroît un bolus à la demande, le bolus ayant une quantité de bolus,
b) un module de collecte de données (1.1, 1.2, 1.3, 1.6, 1.7) destiné à collecter des données pertinentes pour la quantité de bolus, le module de collecte de données (1.1, 1.2, 1.3, 1.6, 1.7) étant fonctionnellement couplé au module d'administration (2.5, 2.8),
c) un module de temporisation (2.7), le module de temporisation (2.7) étant conçu pour déclencher un début d'administration de bolus à un intervalle de temporisation thérapeutiquement significatif après la collecte des données, **caractérisé en ce que** le module de temporisation est réalisé par un circuit ou un code de microprogramme qui vérifie de façon continue si une condition pour terminer l'intervalle de temporisation est respectée et délivre un signal de déclenchement lorsque la condition est respectée, la condition pour terminer l'intervalle de temporisation étant donnée par la réception d'un accusé de réception d'un utilisateur et/ou par un signal délivré par une unité de mesure continue du glucose.

2. Système de perfusion d'insuline ambulatoire selon la revendication 1, **caractérisé par** un module de détermination de bolus (1.8), le module de détermination de bolus étant conçu pour déterminer la quantité de bolus au moins en partie en fonction des données collectées.

3. Système de perfusion d'insuline ambulatoire selon la revendication 2, **caractérisé en ce que** le module de collecte de données (1.1, 1.2, 1.3, 1.6, 1.7) comporte un module de mesure de glycémie (1.7) et le module de détermination de bolus (1.8) est conçu pour déterminer la quantité de bolus au moins en partie en fonction d'une valeur de glycémie mesurée par le module de mesure de glycémie (1.7).

4. Système de perfusion d'insuline ambulatoire selon la revendication 3, **caractérisé en ce que** le module de détermination de bolus (1.8) est conçu pour déterminer, en fonction d'une valeur de glycémie mesurée par le module de mesure de glycémie (1.7), une quantité de bolus de correction d'un bolus de correction et le module d'administration (2.7, 2.8) est conçu pour déclencher un début d'administration de bolus de correction sans temporisation thérapeutiquement significative.

5. Système de perfusion d'insuline ambulatoire selon l'une ou l'autre des revendications 2 à 4, **caractérisé en ce que** le module de collecte de données (1.1, 1.2, 1.3, 1.6, 1.7) comporte une interface utilisateur (1.1, 1.2), l'interface utilisateur (1.1, 1.2) étant conçue pour collecter des données caractéristiques des repas auprès d'un utilisateur, et le module de détermination de bolus (1.8) est conçu pour déterminer la quantité de bolus au moins en partie en fonction des données caractéristiques des repas.

6. Système de perfusion d'insuline ambulatoire selon l'une quelconque des revendications précédentes, **caractérisé en ce que** le module de temporisation (2.7) est conçu pour déclencher le début de l'administration de bolus après un intervalle de temps prédéfini, l'intervalle de temps prédéfini définissant l'intervalle de temporisation.

7. Système de perfusion d'insuline ambulatoire selon l'une quelconque des revendications précédentes dans lequel le module de temporisation (2.7) est configuré pour recevoir, dans l'intervalle de temporisation, un accusé de réception d'un utilisateur, **caractérisé en ce que** le module de temporisation (2.7) est conçu pour annuler un intervalle de temporisation en cours sans déclencher l'administration de bolus si un intervalle de temporisation maximal a expiré sans réception de l'accusé de réception de l'utilisateur.

8. Système de perfusion d'insuline ambulatoire selon l'une quelconque des revendications précédentes, **caractérisé par** un module d'alerte (2.2) afin de générer une alerte pendant que l'intervalle de temporisation est en cours et/ou quand l'intervalle de temporisation est terminé.

9. Système de perfusion d'insuline ambulatoire selon l'une quelconque des revendications précédentes, **caractérisé en ce que** le module de temporisation (2.7) peut être désactivé sélectivement.

10. Système de perfusion d'insuline ambulatoire selon l'une quelconque des revendications précédentes, **caractérisé en ce que** le module d'administration (2.5, 2.8) et le module de collecte de données (1.6) sont contenus dans des boîtiers distincts.

11. Système de perfusion d'insuline ambulatoire selon la revendication 10, **caractérisé en ce que** le module de temporisation (2.7) est disposé dans le même boîtier que le module d'administration (2.5, 2.8).

12. Système de perfusion d'insuline ambulatoire selon l'une quelconque des revendications précédentes, **caractérisé en ce que** le système comporte ou peut être fonctionnellement couplé à une unité de surveillance continue du glucose et est conçu pour tenir compte de valeurs de glycémie mesurées au début et/ou pendant et/ou à la fin de l'intervalle de temporisation pour déterminer la quantité de bolus.

13. Système de perfusion d'insuline ambulatoire selon l'une quelconque des revendications précédentes, **caractérisé en ce que** le module de temporisation (2.7) est conçu pour stocker un intervalle de temporisation minimal, l'intervalle de temporisation minimal étant une limite inférieure pour la longueur de l'intervalle de temporisation.
